(19) Europäisches Patentamt
European Patent Office
Office européen des brevets

(11) EP 0 934 271 B1

(12) **EUROPEAN PATENT SPECIFICATION**

(45) Date of publication and mention of the grant of the patent:
**03.09.2003 Bulletin 2003/36**

(21) Application number: **96936767.1**

(22) Date of filing: **28.10.1996**

(51) Int Cl.[7]: **C07D 211/52**, A61K 31/445

(86) International application number:
**PCT/US96/16822**

(87) International publication number:
**WO 98/018761 (07.05.1998 Gazette 1998/18)**

(54) **SUBSTITUTED ARYLALKYLAMINES AS NEUROKININ ANTAGONISTS**

SUBSTITUIERTE ARYLALKYLAMINE ALS NEUROKININ-ANTAGONISTEN

ARYLALKYLAMINES SUBSTITUES UTILISES COMME ANTAGONISTES DE LA NEUROKININE

(84) Designated Contracting States:
**AT BE CH DE DK ES FI FR GB GR IE IT LI LU NL PT SE**
Designated Extension States:
**LT LV RO**

(43) Date of publication of application:
**11.08.1999 Bulletin 1999/32**

(73) Proprietor: **SCHERING CORPORATION**
**Kenilworth New Jersey 07033 (US)**

(72) Inventors:
- **REICHARD, Gregory, A.**
**Morris Plains, NJ 07950 (US)**
- **ASLANIAN, Robert, G.**
**Rockaway, NJ 07866 (US)**

(74) Representative: **Ritter, Stephen David et al**
**Mathys & Squire**
**100 Gray's Inn Road**
**London WC1X 8AL (GB)**

(56) References cited:
**EP-A- 0 474 561** **WO-A-94/29309**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

### BACKGROUND OF THE INVENTION

The present invention relates to a genus of substituted arylalkylamines useful as antagonists of tachykinin receptors, in particular as antagonists of the neuropeptides neurokinin-1 receptor ($NK_1$) and/or neurokinin-2 receptor ($NK_2$) and/or neurokinin-3 receptor ($NK_3$).

Neurokinin receptors are found in the nervous system and the circulatory system and peripheral tissues of mammals, and therefore are involved in a variety of biological processes. Neurokinin receptor antagonists are consequently expected to be useful in the treatment or prevention of various mammalian disease states, for example asthma, cough, bronchospasm, inflammatory diseases such as arthritis, central nervous system conditions such as migraine and epilepsy, nociception, and various gastrointestinal disorders such as Crohn's disease.

In particular, $NK_1$ receptors have been reported to be involved in microvascular leakage and mucus secretion, and $NK_2$ receptors have been associated with smooth muscle contraction, making $NK_1$ and $NK_2$ receptor antagonists especially useful in the treatment and prevention of asthma.

Some $NK_1$ and $NK_2$ receptor antagonists have previously been disclosed: arylalkylamines were disclosed in U.S. Patent 5,350,852, issued September 27, 1994, and spiro-substituted azacycles were disclosed in WO 94/29309, published December 22, 1994.

### SUMMARY OF THE INVENTION

Compounds of the present invention are represented by the formula I

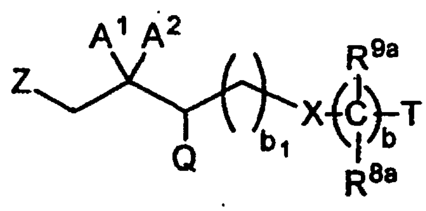

or a pharmaceutically acceptable salt thereof, wherein:

$A^1$ is $-OR^6$, $-N(R^6)(R^7)$, $-S(O)_eR^{13}$, or $-(C(R^6)(R^7))_{1-6}-N(R^6)(R^7)$ and $A^2$ is H, or $A^1$ and $A^2$ together are =O, $=C(R^6)(R^7)$, or $=NOR^6$;

Q is $R^5$-phenyl;

T is $R^4$-aryl;

b is 0, 1 or 2;

$b_1$ is 1 or 2;

X is -O-, $-NR^6-$, $-N(R^6)C(O)-$, $-OC(O)NR^6-$, or $-N(R^6)C(O)O-$;

$R^4$ and $R^5$ are independently 1-3 substituents independently selected from the group consisting of H, halogeno, $-OR^6$, $-OC(O)R^6$, $-OC(O)N(R^6)(R^7)$, $-N(R^6)(R^7)$, $C_{1-6}$ alkyl, $-CF_3$, $-C_2F_5$, $-COR^6$, $-CO_2R^6$, $-CON(R^6)(R^7)$, $-S(O)_eR^{13}$, -CN, $-OCF_3$, $-NR^6CO_2R^{16}$, $-NR^6COR^7$, $-NR^8CON(R^6)(R^7)$, $R^{15}$-phenyl, $R^{15}$-benzyl, $NO_2$, $-N(R^6)S(O)_2R^{13}$ or $-S(O)_2N(R^6)(R^7)$; or adjacent $R^4$ substituents or adjacent $R^5$ substituents can form a $-O-CH_2-O-$ group; and $R^4$ can also be $R^{15}$-heteroaryl;

$R^6$, $R^7$, $R^8$, $R^{8a}$, and $R^{13}$ are independently selected from the group consisting of H, $C_{1-6}$ alkyl, $C_2$-$C_6$ hydroxyalkyl, $C_1$-$C_6$ alkoxy-$C_1$-$C_6$ alkyl, $R^{15}$-phenyl, and $R^{15}$-benzyl; or $R^6$ and $R^7$, together with the nitrogen to which they are attached, form a ring of 5 to 6 members, wherein 0, 1 or 2 ring members are selected from the group consisting of -O-, -S- and $-N(R^{19})-$;

$R^9$ and $R^{9a}$ are independently selected from the group consisting of $R^6$ and $-OR^6$, provided that when $R^9$ is OH, X is $-N(R^6)C(O)-$;

$R^{10}$ is independently selected from the group consisting of H and $C_{1-6}$ alkyl;

$R^{15}$ is 1 to 3 substituents independently selected from the group consisting of H, $C_1$-$C_6$ alkyl, $C_1$-$C_6$ alkoxy, $C_1$-$C_6$ alkylthio, halogeno, $-CF_3$, $-C_2F_5$, $-COR^{10}$, $-CO_2R^{10}$, $-C(O)N(R^{10})_2$, $-S(O)_eR^{10}$, -CN, $-N(R^{10})COR^{10}$, $-N(R^{10})CON(R^{10})_2$ and $-NO_2$;

$R^{16}$ is $C_{1-6}$ alkyl, $R^{15}$-phenyl or $R^{15}$-benzyl;

$R^{19}$ is H, $C_1$-$C_6$ alkyl, $-C(O)N(R^{10})_2$, $-CO_2R^{10}$, $-(C(R^8)(R^9))_f-CO_2R^{10}$ or $-(C(R^8)(R^9))_u-C(O)N(R^{10})_2$;

f is 1-6;
u is 0-6; and
Z is

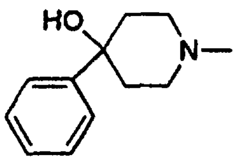

or

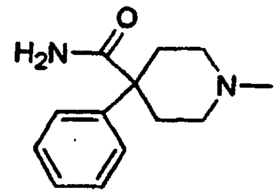

;

wherein:

"aryl" refers to phenyl, naphthyl, indenyl, tetrahydronaphthyl, indanyl, anthracenyl or fluorenyl; and
"heteroaryl" refers to 5- to 10-membered single or benzofused aromatic rings .

comprising 1 to 4 heteroatoms independently selected from the group consisting of -O-, -S- and -N=, provided that the rings do not include adjacent oxygen and/or sulfur atoms.

Preferred are compounds of formula I wherein X is $-NR^6-$ or $-N(R^6)C(O)-$. Also preferred are compounds wherein b is 0 or 1 when X is $-NR^6-$ or $-N(R^6)C(O)-$. Also preferred are compounds wherein $b_1$ is 1. T is preferably $R^4$-phenyl. Also preferred are compounds wherein $R^{8a}$ and $R^{9a}$ are independently hydrogen, $C_2$-$C_6$ hydroxyalkyl or $C_1$-$C_6$-alkoxy-$C_1$-$C_6$-alkyl, with hydrogen being more preferred. Especially preferred are compounds wherein $R^{8a}$ and $R^{9a}$ are each hydrogen, X is $-NR^6-$ of $-N(R^6)C(O)-$, T is $R^4$-aryl and $R^4$ is two substituents selected from $C_1$-$C_6$ alkyl, halogeno, $-CF_3$ and $C_1$-$C_6$ alkoxy.

Especially preferred definitions for Q are $R^5$-phenyl, wherein $R^5$ is preferably two halogeno substrtuents.

Preferred are compounds of formula I wherein $A^1$ is $-OR^6$, $-N(R^6)(R^7)$, $-S(O)_eR^{13}$ or $-(C(R^6)(R^7))_{1\text{-}6}-N(R^6)(R^7)$ and $A^2$ is H; also preferred compounds are where $A^1$ and $A^2$ together are =O, $=C(R^6)(R^7)$ or $=NOR^6$.

This invention also relates to the use of a compound of formula I for the manufacture of a medicament for the treatment of asthma, cough, bronchospasm, inflammatory diseases such as arthritis, central nervous system conditions such as migraine and epilepsy, nociception, and various gastrointestinal disorders such as Crohn's disease.

In another aspect, the invention relates to a pharmaceutical composition comprising a compound of formula I in a pharmaceutically acceptable carrier. The invention also relates to the use of said pharmaceutical composition for the manufacture of a medicament for the treatment of asthma, cough, bronchospasm, inflammatory diseases such as arthritis, migraine, nociception, and various gastrointestinal disorders such as Crohn's disease.

## DETAILED DESCRIPTION

As used herein, the term "alkyl" means straight or branched alkyl chains. "Lower alkyl" refers to alkyl chains of 1-6 carbon atoms and, similarly, lower alkoxy refers to alkoxy chains of 1-6 carbon atoms.

"Aryl" means phenyl, naphthyl, indenyl, tetrahydronaphthyl, indanyl, anthracenyl or fluorenyl.

"Halogeno" refers to fluoro, chloro, bromo or iodo atoms.

"Heterocycloalkyl" refers to tetrahydrofuranyl, pyrrolidinyl, piperidinyl, morpholinyl, thiomorpholinyl and piperazinyl. $R^4$-heterocycloalkyl refers to such groups wherein substitutable ring carbon atoms have an $R^4$ substituent.

"Heteroaryl" refers to 5- to 10-membered single or benzofused aromatic rings comprising 1 to 4 heteroatoms independently selected from the group consisting of -O-, -S- and -N=, provided that the rings do not include adjacent oxygen and/or sulfur atoms. Examples of single-ring heteroaryl groups are pyridyl, oxazolyl, isoxazolyl, oxadiazolyl, furanyl, pyrrolyl, thienyl, imidazolyl, pyrazolyl, tetrazolyl, thiazolyl, isothiazolyl, thiadiazolyl, pyrazinyl, pyrimidyl, pyridazinyl and triazolyl. Examples of benzofused heteroaryl groups are indolyl, quinolyl, benzothienyl (i.e., thianaphthenyl), benzimidazolyl, benzofuranyl, benzoxazolyl and benzofurazanyl. N-oxides of nitrogen-containing heteroaryl groups are also included. All positional isomers are contemplated, e.g., 1-pyridyl, 2-pyridyl, 3-pyridyl and 4-pyridyl. $R^4$-heteroaryl refers to such groups wherein substitutable ring carbon atoms have an $R^4$ substituent.

Where $R^6$ and $R^7$ substituents form a ring and additional heteroatoms are present, the rings do not include adjacent oxygen and/or sulfur atoms or three adjacent heteroatoms. Typical rings so formed are morpholinyl, piperazinyl and piperidinyl.

In the above definitions, wherein variables such as $R^6$, $R^7$, $R^8$, $R^9$, $R^{13}$ and $R^{15}$ are said to be independently selected from a group of substituents, we mean that $R^6$, $R^7$, $R^8$, $R^9$, $R^{13}$ and $R^{15}$ are independently selected, but also

that where an $R^6$, $R^7$, $R^8$, $R^9$, $R^{13}$ or $R^{15}$ variable occurs more than once in a molecule, those occurrences are independently selected (e.g., if $R^4$ is -$OR^6$ wherein $R^6$ is methyl, X can be -N($R^6$)- wherein $R^6$ is ethyl). Similarly, $R^4$ and $R^5$ can be independently selected from a group of substituents, and where more than one $R^4$ and $R^5$ are present, the substitutents are independently selected; those skilled in the art will recognize that the size and nature of the substituent (s) will affect the number of substituents which can be present.

Compounds of the invention can have at least one asymmetrical carbon atom and therefore all isomers, including diastereomers, enantiomers and rotational isomers are contemplated as being part of this invention. The invention includes d and l isomers in both pure form and in admixture, including racemic mixtures. Isomers can be prepared using conventional techniques, either by reacting optically pure or optically enriched starting materials or by separating isomers of a compound of formula I.

Those skilled in the art will appreciate that for some compounds of formula I, one isomer will show greater pharmacological activity than other isomers.

Compounds of the invention have at least one amino group which can form pharmaceutically acceptable salts with organic and inorganic acids. Examples of suitable acids for salt formation are hydrochloric, sulfuric, phosphoric, acetic, citric, oxalic, malonic, salicylic, malic, fumaric, succinic, ascorbic, maleic, methanesulfonic and other mineral and carboxylic acids well known to those in the art. The salt is prepared by contacting the free base form with a sufficient amount of the desired acid to produce a salt. The free base form may be regenerated by treating the salt with a suitable dilute aqueous base solution such as dilute aqueous sodium bicarbonate. The free base form differs from its respective salt form somewhat in certain physical properties, such as solubility in polar solvents, but the salt is otherwise equivalent to its respective free base forms for purposes of the invention.

Certain compounds of the invention are acidic (e.g., those compounds which possess a carboxyl group). These compounds form pharmaceutically acceptable salts with inorganic and organic bases. Examples of such salts are the sodium, potassium, calcium, aluminum, gold and silver salts. Also included are salts formed with pharmaceutically acceptable amines such as ammonia, alkyl amines, hydroxyalkylamines, N-methylglucamine and the like.

It will be appreciated that compounds of formula I can be prepared using methods well known to those skilled in the art. The following illustrates typical procedures for preparing various compounds. It will be understood that compounds marked with an asterisk (*) are not within the scope of the invention, but are provided by way of illustrating analogous procedures that may be used to synthesise compounds of the invention.

<u>Procedure A</u>:

Compounds of formula I wherein X is -C(O)-*, -O-, -$S(O)_e$-*, -C(O)N($R^6$)-*, -OC(O)$NR^6$-, -OC(=S)$NR^6$-*, -C (=$NOR^6$)-*, -$S(O)_2$N($R^6$)-* or -OC(O)-*, Q is $R^5$ phenyl, $b_1$ is 1 or 2, and the remaining variables are as defined above, can be prepared as shown in the following reaction scheme:

Step 1:

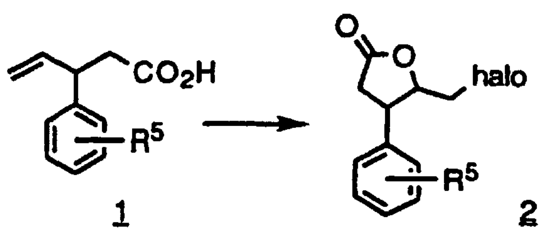

1 2

In step 1, compound 1, wherein $R^5$ is as defined above, is treated with a halogenating agent such as $I_2$ or N-bromosuccinimide in an organic solvent such as $CH_3CN$, THF or DMF at a temperature in the range of 0 to 25°C to give the halolactone 2.

Step 2:

2 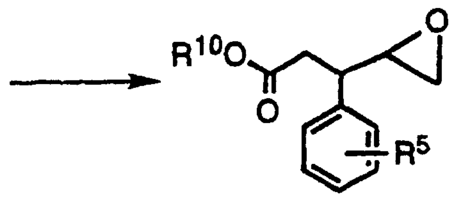 3

In step 2, compound 2 is dissolved in an alcohol $R^{10}OH$ wherein $R^{10}$ is preferably methyl. A base such as $Cs_2CO_3$ or $Na_2CO_3$ is added and the mixture stirred at a temperature range of 0 to 50°C to give the epoxide 3.

Alternatively, a lower alkyl ester of 1 can be epoxidized by a suitable epoxidizing agent such as dimethyl dioxirane or m-CPBA to obtain a compound of formula 3.

Step 3:

3 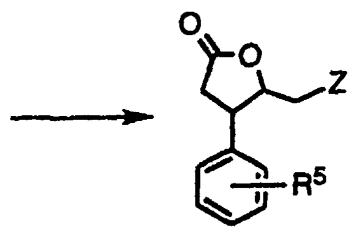 4

In step 3, a solution of epoxide 3 in an alcohol such as $CH_3OH$, $CH_3CH_2OH$, or more preferably $CF_3CH_2OH$, is treated with a secondary amine Z nucleophile where $R^4$ is as defined above, at 0 to 90°C to give the lactone 4.

Step 4:

4 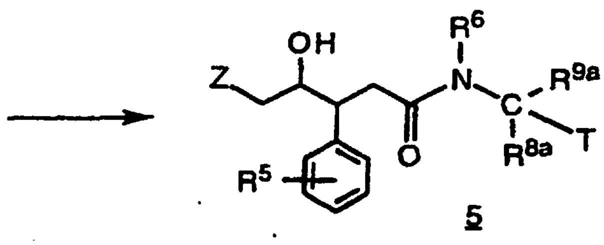

5

For compounds where X is -C(O)N($R^6$)-* and $b_1$ is 1, lactone 4 is treated with the corresponding dialkylamine -N($R^6$) C($R^{9a}$)($R^{8a}$)-T in an alcohol such as $CH_3OH$, $CH_3CH_2OH$, or more preferably $CF_3CH_2OH$, at 0 to 90°C to give the amide 5. Compounds of formula 5 can be converted to the corresponding keto compounds (wherein $A^1$ and $A^2$ together are =O) by oxidation with a suitable reagent such as pyridinium dichromate, Dess Martin reagent, Jones reagent, TPAP, or Swem oxidation; the keto compounds are converted to the corresponding oximes (compounds wherein $A^1$ and $A^2$ together are =NOR$^6$) by treatment of the keto compound with hydroxyl amine or an appropriate alkoxyl amine in pyridine at 23°C to 80 °C. Accordingly, the corresponding olefins (compounds wherein $A^1$ and $A^2$ together are =C($R^6$)($R^7$)) can be prepared from the respective keto compounds by using standard Wittig chemistry known to those skilled in the art.

Step 4a:

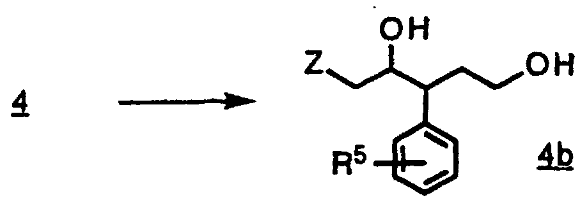

For compounds where X is -C(O)-*, -O-, -$S(O)_e$-*, -OC(O)$NR^6$-, -OC(=S)$NR^6$-*, -C(=$NOR^6$)-*, -$S(O)_2N(R^6)$-* or -OC(O)-* and $b_1$ is 2, lactone 4 is treated with an appropriate reducing agent such as Dibal-H, LAH, $LiBH_4$ or $NaBH_4$, with a temperature in the range of -78°C to 80°C to give the corresponding diol 4b. With appropriate protection of reactive groups, compounds of formula 4b can be converted to compounds where X is -C(O)-*, -O-, -$S(O)_e$-*, -OC(O)$NR^6$-, -OC(=S)$NR^6$-*, -C(=$NOR^6$)-*, -$S(O)_2N(R^6)$-* or -OC(O)-*by appropriate functional group interchange or functionalization of the terminal alcohol group. The corresponding keto compounds (compounds wherein $A^1$ and $A^2$ together are =O) may be prepared by oxidation with a suitable reagent such as pyridinium dichromate, Dess Martin reagent, Jones reagent, TPAP or Swem oxidation; synthesis of the corresponding oximes (compounds wherein $A^1$ and $A^2$ together are =$NOR^6$) are made by treatment of the keto compound with hydroxyl amine or an appropriate alkoxyl amine in pyridine at 23°C to 80 °C. Accordingly, the corresponding olefins (compounds wherein $A^1$ and $A^2$ together are =$C(R^6)(R^7)$) can be prepared from the respective keto compounds by using standard Wittig chemistry known to those skilled in the art.

Procedure B:

Compounds of formula I wherein X is -$NR^6$-, -$NR^6$C(O)-, -$N(R^6)$C(=S)O-*, -$N(R^6)S(O)_2$-*, -$N(R^6)$C(O)O- or -$N(R^6)$C(O)$N(R^7)$-*, Q is $R^5$ phenyl, $b_1$ is 1 and the remaining variables are as defined above, can be prepared as shown in the following reaction scheme:

Step 1:

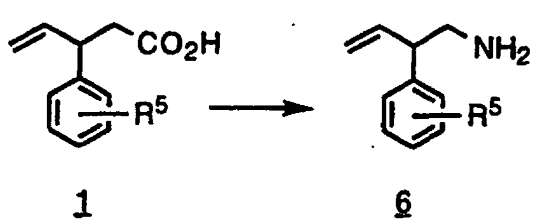

In step 1, the acid 1 is subjected to conditions typical of a Curtius rearrangement, for example; treatment with diphenylphosphoryl azide and a suitable base such as triethyl amine ($Et_3N$) in an appropriate solvent such as *t*-butanol. After heating to reflux, cooling and appropriate purification such as recrystallization or silica gel chromatography, the corresponding N-Boc protected amine of compound 6 is isolated. Deprotection of the Boc group by standard conditions known to those skilled in the art, such as treatment with an acid such as hydrochloric acid or trifluoroacetic acid, provides compound 6.

Step 2:

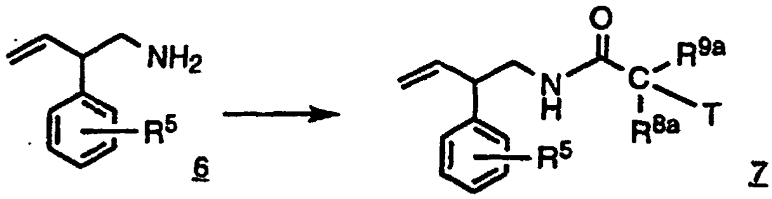

6 7

In step 2, amine 6 is acylated by standard procedures, for example by treatment with an acid chloride of the formula T-C($R^{9a}$)($R^{8a}$)COCl, wherein T, $R^{8a}$ and $R^{9a}$ are as defined as above, in the presence of an amine base in an inert organic solvent such as $CH_2Cl_2$ or toluene, preferably $CH_2Cl_2$, at a temperature of from -10 to 50°C. Suitable bases include $(CH_3)_3N$, $Et_3N$ and pyridine, preferably $Et_3N$. Other acylating agents such as anhydrides are also suitable. Other coupling methods known to those skilled in the art, such as EDC coupling, may also be employed. Correspondingly, for the preparation of compounds wherein X is -N($R^6$)S(O)$_2$-*, -N($R^6$)C(O)O- or -N($R^6$)C(O)N($R^7$)-*, the amine is treated with the appropriate sulfonyl halide, chloroformate, or isocyanate respectively.

Step 3:

Step 3:

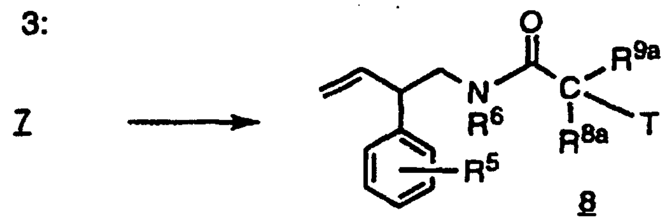

7 8

In step 2, compound 7 is treated with a base such as NaH or LDA, in an inert organic solvent such as THF, ether, DMSO or DMF, preferably THF. The resulting anion is treated with an alkylating agent $R^6$L, wherein R6 is as defined above and L is a suitable leaving group such as Cl, Br, I, triflate or mesylate, to give the product of formula 8. The reactions are typically run at 0 to 50°C.

Step 4:

Step 4:

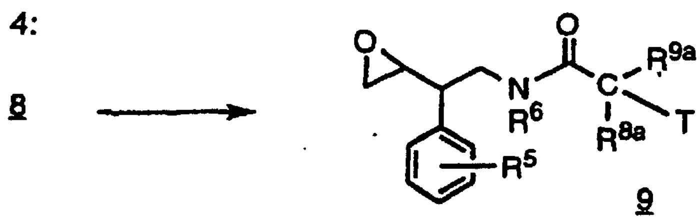

8 9

In step 3, compound 8 is oxidized to the epoxide 9 by treatment with an oxidizing agent such as dimethyl dioxirane in an inert organic solvent such as acetone at a temperature of 0 to 30°C. Other suitable oxidants can be used, for example m-CPBA in a solvent such as $CH_2Cl_2$. Suitable protective groups on $R^{9a}$, $R^{8a}$, and T on moieties susceptible to oxidation under these conditions may be necessary.

Step 5:

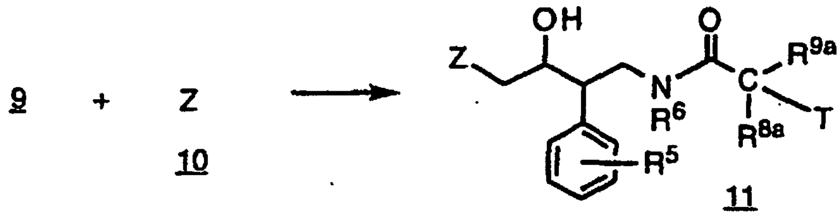

9 + Z 10 → 11

In step 5, the epoxide of formula 9 is converted to the amine of formula 11 by treating a solution of epoxide 9 in an alcohol such as $CH_3OH$, $CH_3CH_2OH$, or more preferably $CF_3CH_2OH$, with a secondary amine Z nucleophile, 10 as defined above, at 0 to 90°C to give the amino alcohol of formula 11. Compounds of formula 11 can be converted to the corresponding keto (compounds wherein $A^1$ and $A^2$ together are =O) then to the corresponding oximes (compounds wherein $A^1$ and $A^2$ together are $=NOR^6$) as described above in Procedure A. Accordingly, the corresponding olefins (compounds wherein $A^1$ and $A^2$ together are $=C(R^6)(R^7)$) may be prepared from the keto compounds using standard Wittig chemistry known to those skilled in the art.

Procedure C:

Step 1:

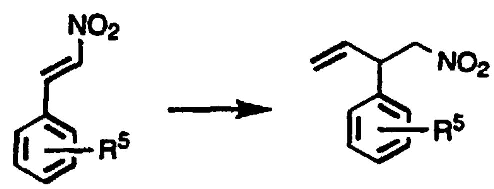

12 13

For compounds where X is a bond, -C(O)-*, -O-, $-NR^6-$, $-S(O)_e-$*, $-N(R^6)C(O)-$, $-C(O)N(R^6)-$*,$-OC(O)NR^6-$, -OC(=S) $NR^6$-*,$-N(R^6)C(=S)O-$*, $-C(=NOR^6)-$*, $-S(O)_2N(R^6)-$*, $-N(R^6)S(O)_2-$*, $-N(R^6)C(O)O-$ or -OC(O)-*and $b_1$ is 1, the nitro olefin 12 is added to a mixture of a copper salt, preferably CuCN, and vinyl magnesium bromide in a suitable solvent, preferably THF, with a temperature range of -78 °C to 0 °C, to give after workup and appropriate purification the nitro product 13. This product can be reduced to give the primary amine 6 or the nitro group may be transformed to the corresponding carboxylic acid via a standard Nef reaction, then to the primary alcohol with functional group transformations known to those skilled in the art. Such compounds can be converted to compounds where X is -C(O)-*, -O-, $S(O)_e$-*, $-OC(O)NR^6-$, $OC(=S)NR^6$-*, $-C(=NOR^6)-$*, $-S(O)_2N(R^6)-$* or -OC(O)-* by appropriate functional group interchange or functionalization of the terminal alcohol group. The corresponding keto compounds (i.e., compounds wherein $A^1$ and $A^2$ together are =O) may be prepared by oxidation with a suitable reagents such as pyridinium dichromate, Dess Martin reagent, Jones reagent, TPAP, or Swem oxidation; synthesis of the corresponding oximes (i.e., compounds wherein $A^1$ and $A^2$ together are $=NOR^6$) are made by treatment of the keto compound with hydroxyl amine or an appropriate alkoxyl amine in pyridine at 23°C to 80 °C. Accordingly, the corresponding olefins (compounds wherein $A^1$ and $A^2$ together are $=C(R^6)(R^7)$) can be prepared from the respective keto compounds by using standard Wittig chemistry known to those skilled in the art.

Reactive groups not involved in the above processes can be protected during the reactions with conventional protecting groups which can be removed by standard procedures after the reaction. The following Table 1 shows some typical protecting groups:

**Table 1**

| Group to be Protected | Group to be Protected and Protecting Group |
|---|---|
| $-COOH$ | $-COOalkyl$, $-COObenzyl$, $-COOphenyl$ |
| >NH | >NCOalkyl, >NCObenzyl, >NCOphenyl, >$NCH_2OCH_2CH_2Si(CH_3)_3$ >$NC(O)OC(CH_3)_3$, >N-benzyl, >$NSi(CH_3)_3$, 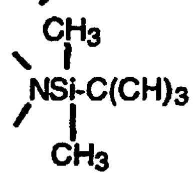 |
| $-NH_2$ | 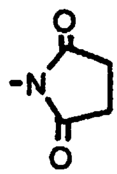 |
| $-OH$ | $-OCH_3$, $-OCH_2OCH_3$, $-OSi(CH_3)_3$, 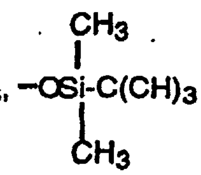 or $-OCH_2phenyl$ |

Compounds of formula I have been found to be antagonists of $NK_1$ and/or $NK_2$ and/or $NK_3$ receptors, and are therefore useful in treating conditions caused or aggravated by the activity of said receptors.

The present invention also relates to a pharmaceutical composition comprising a compound of formula I and a pharmaceutically acceptable carrier. Compounds of this invention can be administered in conventional oral dosage forms such as capsules, tablets, powders, cachets, suspensions or solutions, or in injectable dosage forms such as solutions, suspensions, or powders for reconstitution The pharmaceutical compositions can be prepared with conventional excipients and additives, using well known pharmaceutical formulation techniques. Pharmaceutically acceptable excipients and additives include non-toxic and chemically compatibile fillers, binders, disintegrants, buffers, preservatives, anti-oxidants, lubricants, flavorings, thickeners, coloring agents, emulsifiers and the like.

The daily dose of a compound of formula I for treating asthma, cough, bronchspasm, inflammatory diseases, migraine, nociception and gastrointestinal disorders is about 0.1 mg to about 20 mg/kg of body weight per day, preferably about 0.5 to about 15 mg/kg. For an average body weight of 70 kg, the dosage range is therefore from about 1 to about 1500 mg of drug per day, preferably about 50 to about 200 mg, more preferably about 50 to about 500 mg/kg per day, given in a single dose or 2-4 divided doses. The exact dose, however, is determined by the attending clinician and is dependent on the potency of the compound administered, the age, weight, condition and response of the patient.

Following are examples of preparing starting materials and compounds of formula I. Compounds marked with an asterisk (*) are not within the scope of the present invention, but are provided to illustrate analogous procedures by which compounds of the invention may be synthesised.

**Example 1**

*N*-[2-(3,4-dichlorophenyl)-3-hydroxy-4-(4-hydroxy-4-phenyl-1-piperidinyl)butyl]-*N*-methyl benzamide

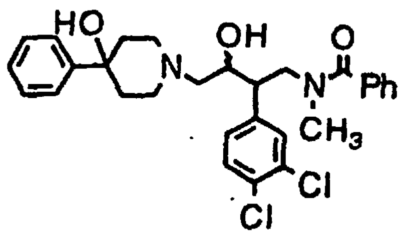

Step 1: Cool a solution of 3-(3,4-dichlorophenyl)-2-propeneoic acid (100 g, 461 mmol) in dry DMF (500 mL) to 0°C and treat with $Cs_2CO_3$ (100 g, 307 mmol, 0.66 eq). Stir the resulting off-white slurry for 15 min, then add $CH_3I$ (33 mL, 530 mmol, 1.15 eq) via syringe. After 1 h, add additional DMF (250 mL), stir the slurry for 14 h and partition between EtOAc (1.5 L) and half saturated aqueous $NaHCO_3$ (500 mL). Separate the organic layer and extract the aqueous layer twice with EtOAc (1 L, 500 mL). Wash the combined organic layers with half saturated aqueous $NaHCO_3$ (500 mL) and water (5 x 500 mL), then dry ($Na_2SO_4$) and concentrate to obtain 105.4 g (456 mmol, 99%) of methyl 3-(3,4-dichlorophenyl)-2-propenoate as light brown needles.

Step 2: Treat a solution of the product of Step 1 (15 g, 65 mmol) in dry THF (250 mL), kept cool in a large ambient temperature water bath, with Dibal-H (140 mL, 140 mmol, 2.15 eq) over 30 min. Stir the resulting solution for 30 min at 23 °C, pour into $Et_2O$ (500 mL), treat with water (5 mL), 15 % NaOH (5 mL) and water (15 mL). Stir for 5 min, dilute the mixture with $Et_2O$ (200 mL) and treat with 15 % NaOH (15 mL). Add $MgSO_4$ to cause a colorless precipitate. Remove the aluminum salts by filtration through a course glass frit. Wash the solids with $Et_2O$ (1 L) and concentrate the filtrate *in vacuo* to give 13.2 g (65 mmol, 99%) of 3-(3,4-dichlorophenyl)-2-propene-1-ol as an off-white solid.

Step 3: Treat a solution of the product of step 2 (13.2 g, 65 mmol) in $CH_2Cl_2$ (250 mL) at 0 °C with pyridine (7.89 mL, 97.5 mmol, 1.5 eq) and dimethylaminopyridine (397 mg, 3.25 0.05 eq), followed by $CH_3COCl$ (6.48 mL, 74.75 mmol, 1.15 eq). Allow the mixture to warm to 23 °C, pour into 1 M HCl (100 mL) and wash the resulting organic layer again with 1 M HCl (100 mL), followed by water (5 x 100 mL; pH=6.5-7). Dry the organic layer ($Na_2SO_4$) and concentrate to obtain 15.4 g (62.9 mmol, 97%) of 3-(3,4-dichlorophenyl)-2-propene-1-ol acetate as a colorless oil.

Step 4: Treat a solution of the product of step 3 (15 g, 61 mmol, dried by azeotropic distillation with toluene, 1 x 50 mL) in dry THF (250 mL) at -78°C with chlorotriethylsilane (20.2 mL, 120 mmol, 2.0 eq) rapidly followed by the addition of potassium bis(trimethylsilyl)amide (183 mL, 91.5 mmol, 1.5 eq of 0.5 M in toluene) via addition funnel over 50 min. Allow the mixture to warm to 23°C and heat to reflux for 3 h. Gradually cool the solution overnight, then quench with saturated $NH_4Cl$ (150 mL). Stir the resultant mixture vigorously for 3h, treat with 1M HCl (150 mL) and then extract with $Et_2O$ (500 mL). Extract the aqueous layer with $Et_2O$ (400 mL), wash the combined organic layers with 5% NaOH (300 mL) and extract with 5 % NaOH (8 x 150 mL). Cool the combined aqueous layers to 5°C and, maintaining the temperature at 5-10 °C, carefully acidify with concentrated HCl (ca 175 mL) to pH 1. Extract the aqueous layer with $CH_2Cl_2$ (2 x 800 mL), dry ($Na_2SO_4$) and concentrate to give 13.4 g (54.5 mmol, 89%) of 3-(3,4-dichlorophenyl)-4-pentenoic acid as a faint yellow oil.

Step 5: Treat a solution of the product of step 4 (13.75 g, 56 mmol, dried by azeotropic distillation with toluene, 100 mL) in dry, freshly distilled *t*-butanol (250 mL) with freshly distilled $Et_3N$ (9.34 mL, 70 mmol, 1.25 eq) followed by diphenylphosphoryl azide (15.1 mL, 70 mmol, 1.25 eq). Heat the resulting solution to reflux for 24 h, cool and concentrate *in vacuo*. Treat the resultant product with toluene (100 mL), concentrate (2 x), dissolve in hexane:EtOAc (1:1) and filter through a pad of silica gel (4 x 10 cm), eluting with hexane:EtOAc (1:1) (1 L). Concentrate the filtrate to obtain 20.7 g of crude 1,1-dimethylethyl-[2-(3,4-dichlorophenyl)-3-butenyl] carbamate.

Step 6: Treat a solution of the product of step 5 (5.32 g of ca 88% pure, 14.8 mmol) in $CH_2Cl_2$ (100 mL) with trifluoroacetic acid (10 mL) and stir for 2 h at 23°C. Treat the mixture with heptane (50 mL) and concentrate *in vacuo*. Take up the resulting crude product in hexane:EtOAc (1:1) and apply to a pad of silica gel (4 x 10 cm) packed with hexane:EtOAc (1:1). Wash the plug with the same solvent (1 L) and then elute the desired product with $CH_2Cl_2$:$CH_3OH$ (saturated with ammonia) (9:1) (1.5 L). Combine the product washes and concentrate to give 3.9 g crude amine used in the next step without further purification.

Step 7: Cool a solution of the product of step 6 (14.8 mmol) in CH2Cl2 (100 mL) to 0°C and treat with $Et_3N$

(3.5 mL, 25.2 mmol, 1.5 eq) and benzoyl chloride (2.1 mL, 17.6 mmol, 1.05 eq). After 10 min, dilute the mixture to 150 mL with CH2Cl2 and wash with 10 % aqueous citric acid (50 mL), water (50 mL) and aqueous saturated NaHCO3 (50 mL), then dry (Na2SO4) and concentrate. Triturate the resulting crude off-white solid with hexane (40 mL) to give 3.29 g (10 mmol, 68%, over three steps) of *N*-[2-(3,4-dichlorophenyl)-3-butenyl] benzamide as a colorless solid.

Step 8: Wash a suspension of NaH (312 mg of 60% in mineral oil, 7.81 mmol, 1.25 eq) in hexane with dry pentane (2 x 100 mL), suspend in dry THF (30 mL) and treat with the product of step 7 (2.0g, 6.25 mmol) at 23°C. Stir the resulting yellow suspension for 20 min at 23°C, then add $CH_3I$ (777 µL, 12.5 mmol, 2.0 eq). After 1 h, pour the mixture onto a pad of silica gel packed with hexane:EtOAc (1:1) (500 mL)and concentrate the filtrate to give 2.1 g (6.25 mmol, >99%) of *N*-[2-(3,4-dichlorophenyl)-3-butenyl]-*N*-methyl benzamide as a light yellow liquid.

Step 9: Treat a solution of the product of step 8 (2.1 g, 6.25 mmol) in dry $CH_2Cl_2$ (50 mL) with a freshly prepared solution of dimethyldioxirane in acetone (100 mL of ca 0.08 M in acetone). Stir the solution for 20 h, concentrate *in vacuo,* azeotrope with toluene (2 x 75 mL) and then purify by silica gel chromatography (column: 4 x 16 cm; eluant: hexane:EtOAc (1:1), to obtain isomer A: 854 mg (2.44 mmol, 39%) of (*trans*)-*N*-[2-(3,4-dichlorophenyl)-2-oxiranylethyl]-*N*-methyl benzamide as a colorless oil; and isomer B: 1.04 g (2.98 mmol, 48%) of (*cis*)-*N*-[2-(3,4-dichlorophenyl)-2-oxiranylethyl]-*N*-methyl benzamide as a colorless solid (total yield 87%). Step 10: Treat a solution of isomer A of step 9 (201 mg, 0.574 mmol) in 2,2,2 trifluoroethanol (3 ml) with 4-hydroxy-4-phenyl piperidine (508 mg, 2.87 mmol, 5 eq). Stir the resulting light yellow solution for 24 h at 23°C, concentrate *in vacuo*, azeotrope with toluene (2 x 5 ml) and concentrate. Purify the resulting crude solid by silica gel chromatography (column: 2.5 x 18 cm; eluant: gradient $CH_2Cl_2$:$CH_3OH$ (saturated with ammonia) (97:3) to (95:5)) to obtain 302.8 mg (0.574 mmol, >99% of the title compound as a colorless foam. HRMS (FAB, M+H$^+$): *m*/*e* calc'd for $[C_{29}H_{33}Cl_2N_2O_3]^+$: 527.1868, found 527.1853.

The compounds of Examples 2-4 are prepared by methods similar to those described in Example 1. For Examples 3-4, the starting material is 3-(4-methoxyphenyl)-4-pentenoic acid, prepared from octyl-3-(4-methoxy)-2-propenopate in a manner similar to the procedure described in Example 1, steps 2-4.

**Example 2**

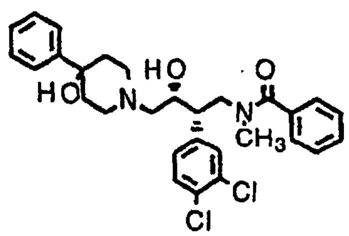

HRMS (FAB, M+H$^+$): *m*/*e* calc'd for $[C_{29}H_{33}Cl_2N_2O_3]^+$: 527.1868, found 527.1863. (Stereochemistry shown is relative.)

**Example 3**

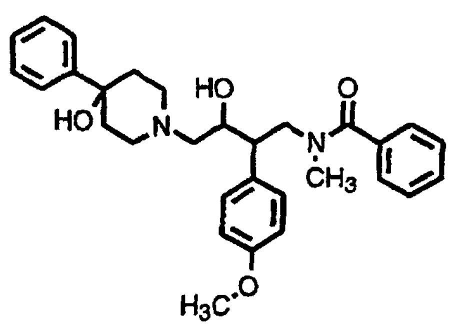

HRMS (FAB, M+H$^+$): *m*/*e* calc'd for $[C_{30}H_{36}N_2O_4]^+$: 489.2753, found 489.2754.

**Example 4**

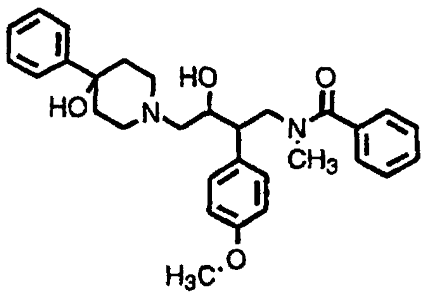

HRMS (FAB, M+H+): *m/e* calc'd for $[C_{30}H_{36}N_2O_4]^+$: 489.2753, found 489.2735.

**Example 5**

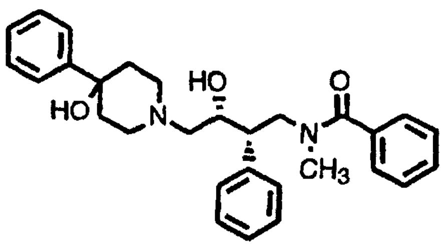

Step 1: Cool a suspension of CuCN (3.3 g, 36.6 mmol, 1.1 eq) in dry THF under argon to -78°C and treat with vinyl magnesium bromide (73.8 mL of 1 M solution in THF, 78 mmol, 2.2eq) dropwise over a period of 30 min. Warm the mixture to 0°C. After stirring for 10 min at 0°C, cool the solution to -20°C, stir for 10 min, then add a solution of *trans*-β-nitrostyrene (5 g, 33.5 mmol) in dry THF (15 mL). Stir the suspension for 1 h, then pour into a 1:2 mixture of 0.1 M HCl / acetic acid (600 mL). Extract the resulting aqueous phase with $CH_2Cl_2$ (400 mL), wash the organic layers with water (2 x 300 mL), dry ($Na_2SO_4$) and concentrate to give 7 g of crude product. Purify by silical gel chromatography (7 x 16 cm, eluant: hexane/$CH_2Cl_2$ (3:1) (1L) gradient to (2:1)) to obtain 2.5 g (14.1 mmol, 42%) of the desired product as a light yellow liquid.

Step 2: Shake aluminum strips (5 g) with a solution of 2% aqueous $HgCl_2$ (60 mL) for 1.5 min. Decant the aqueous layer, wash the foil with ethanol (2 x 50 mL) followed by ether (2 x 50 mL), and suspend in ether (50 mL)/THF (30 mL). Add the product of step 1 (2.5 g) as a solution in THF (20 mL). Add water (5 mL) and $CH_3OH$ (5 mL) and stir the suspension for 48 h at 23°C. Filter the resulting suspension through a cake of celite (10 x 3.5 cm), rinsing with $CH_3OH$. Concentrate the filtrate to obtain 2.1 g (14.1 mol, >95 %) of 2-(3,4-dichlorophenyl)-3-butenyl amine as a light yellow oil.

Step3: Use the product of step 2 in a procedure similar to that described in Example 1, steps 7-10, to obtain the title compound (cis isomer):

HRMS (FAB, M+H+): *m/e* calc'd for $[C_{29}H_{35}N_2O_3]^+$: 459.2648, found 459.2643.

**Example 6**

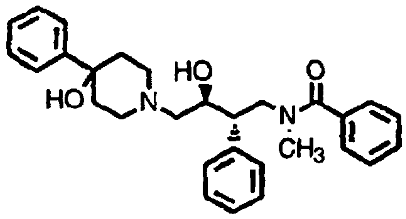

Isolate the trans isomer prepared by the process described in Example 5: HRMS (FAB, M+H+): *m*/*e* calc'd for $[C_{29}H_{35}N_2O_3]^+$: 459.2648, found 459.2644.
Examples 7 and 8 (diastereomers) are prepared in a similar manner to that described in Example 5 using 4-chloro-*trans*-β-nitrostyrene as the starting material.

**Example 7**

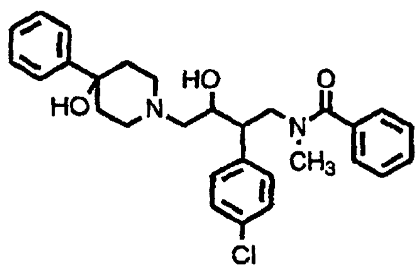

HRMS (FAB, M+H+): *m*/*e* calc'd for $[C_{29}H_{33}Cl_2N_2O_3]^+$: 493.2258, found 493.2261.

**Example 8**

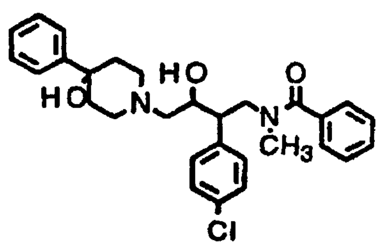

HRMS (FAB, M+H+): *m*/*e* calc'd for $[C_{29}H_{33}ClN_2O_3)^+$: 493.2258, found 493.2270.
Examples 9 and 10 were prepared by a procedure similar to that of Example 5 using 4-methyl-*trans*-β-nitrostyrene as the starting material.

**Example 9**

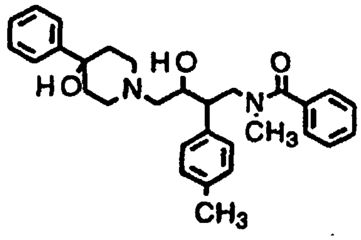

HRMS (FAB, M+H+): *m/e* calc'd for $[C_{30}H_{36}N_2O_3]^+$: 473.2804, found 473.2803.

**Example 10**

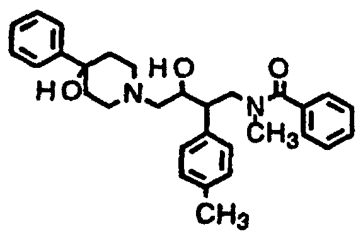

HRMS (FAB, M+H+): *m/e* calc'd for $[C_{30}H_{36}N_2O_3]^+$: 473.2804, found 473.2798.

**Example 11***

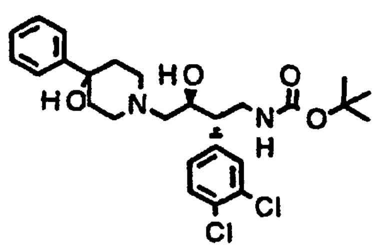

Using 1,1-dimethylethyl-[2-(3,4-dichlorophenyl)-3-butenyl] carbamate as the starting material, carry out the process described in Example 1, steps 9-10, to obtain the title compound. HRMS (FAB, M+H+): *m/e* calc'd for $[C_{26}H_{34}Cl_2N_2O_4]^+$: 509.1974, found 509.1968.

**Example 12***

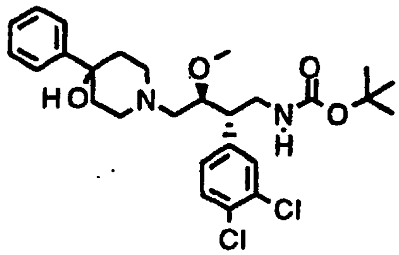

Treat the product of Example 11 in a manner similar to that described in Example 1, step 8 to obtain the title compound.
HRMS (FAB, M+H+): *m/e* calc'd for $[C_{27}H_{36}Cl_2N_2O_4]^+$: 523.2130, found 523.2136.

Examples 13, 14 and 15 are prepared from Examples 1, 2 and 2, respectively, using a procedure similar to that of Example 1, step 8.

**Example 13**

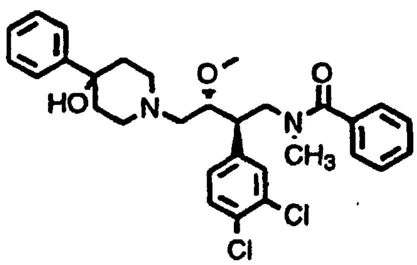

HRMS (FAB, M+H+): *m/e* calc'd for $[C_{30}H_{34}Cl_2N_2O_3]^+$: 541.2025, found 541.2040.

**Example 14**

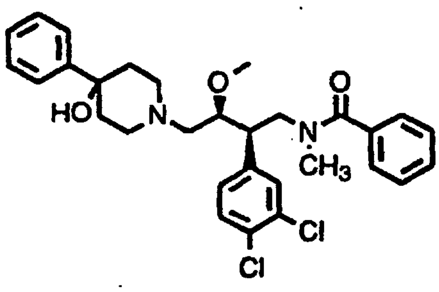

HRMS (FAB, M+H+): *m/e* calc'd for $[C_{30}H_{34}Cl_2N_2O_3]^+$: 541.2025, found 541.2037.

**Example 15***

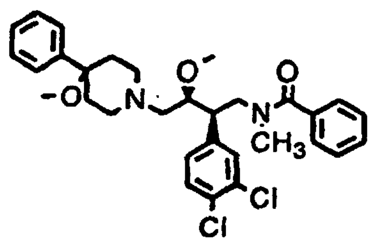

HRMS (FAB, M+H$^+$): *m/e* calc'd for $[C_{31}H_{36}Cl_2N_2O_3]^+$: 555.2181, found 555.2181.

**Example 16**

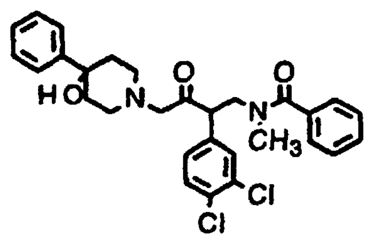

Treat the product of Example 1 in acetone with Jones reagent and stir at 0°C for 1 h. Extract the product with $CH_2Cl_2$ and purify by silica gel chromatography to obtain the title compound. MS : *m/e* 525 (FAB, M+H$^+$).

Examples 17 and 18, regioisomers of the oxime ether, are prepared by heating the product of Example 16 in pyridine with O-methoxylamine HCl at 60°C for 30 min. After removing the pyridine in vacuo, the crude product is purified on a silica gel column.

**Example 17**

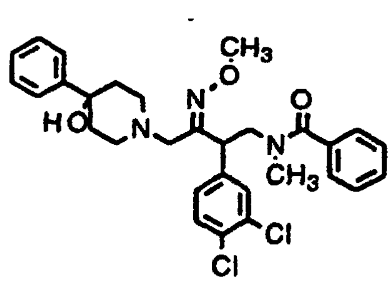

HRMS (FAB, M+H$^+$): *m/e* calc'd for $[C_{30}H_{33}Cl_2N_3O_3]^+$: 554.1977, found 554.1985.

**Example 18**

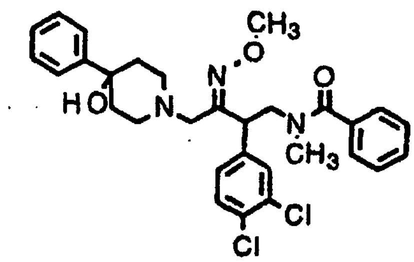

HRMS (FAB, M+H+): *m/e* calc'd for $[C_{30}H_{33}Cl_2N_3O_3]^+$: 554.1977, found 554.1979.

Examples 19, 20, 21 and 22 are prepared from Examples 1, 2, 5 and 6, respectively, using a procedure similar to that described in Example 1, step 8, but using 3,5-(bistrifluoromethyl)benzyl bromide as the alkylhalide.

**Example 19**

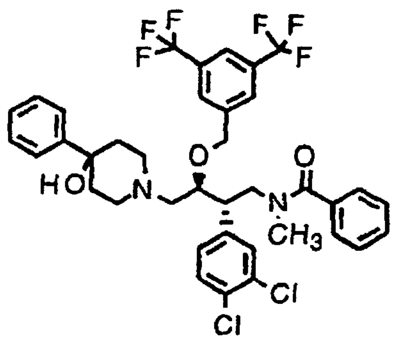

HRMS (FAB, M+H+): *m/e* calc'd for $[C_{38}H_{37}Cl_2F_6N_2O_3]^+$: 753.2085, found 753.2058.

**Example 20**

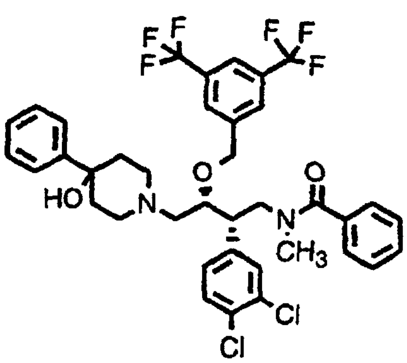

HRMS (FAB, M+H+): *m/e* calc'd for $[C_{38}H_{37}Cl_2F_6N_2O_3]^+$: 753.2085, found 753.2065.

**Example 21**

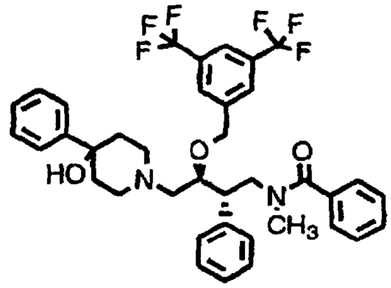

HRMS (FAB, M+H$^+$): *m/e* calc'd for $[C_{38}H_{39}F_6N_2O_3]^+$: 685.2865, found 685.2851

**Example 22**

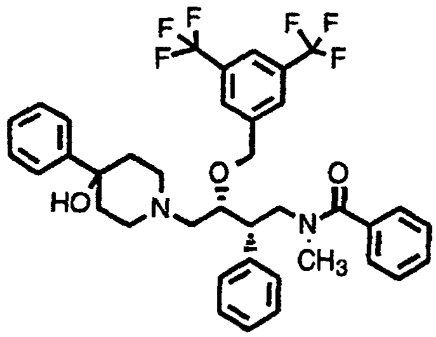

HRMS (FAB, M+H$^+$): *m/e* calc'd for $[C_{38}H_{39}F_6N_2O_3]^+$: 685.2865, found 685.2864.

Examples 23 and 24 are prepared from Examples 1 and 2, respectively, by stirring the amide in dry THF with $LiAlH_4$ for 30 min. at 23°C, partitioning between $Et_2O$, water and NaOH, removing the aluminum salts by filtration, and filtering through a plug of silica gel.

**Example 23**

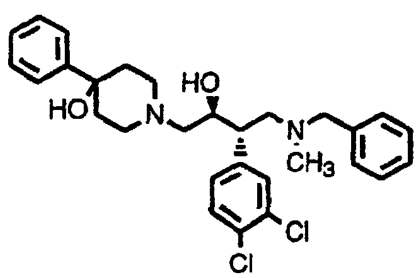

HRMS (FAB, M+H$^+$): *m/e* calc'd for $[C_{29}H_{35}Cl_2N_2O_2]^+$: 513.2076, found 513.2069.

**Example 24**

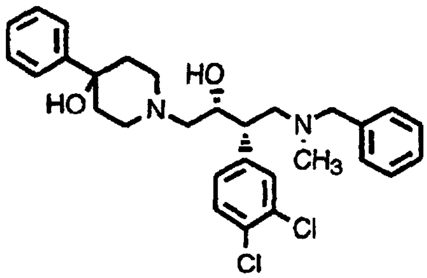

HRMS (FAB, M+H$^+$): *m/e* calc'd for $[C_{29}H_{35}Cl_2N_2O_2]^+$: 513.2076, found 513.2058.

Examples 25 and 26 were prepared from Examples 19 and 20, respectively, using a procedure similar to that used for Examples 23 and 24, using borane-dimethyl sulfide as the reductant.

**Example 25**

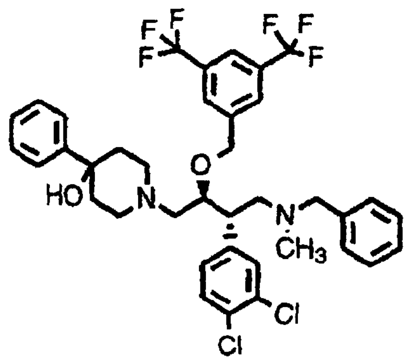

HRMS (FAB, M+H$^+$): *m/e* calc'd for $[C_{38}H_{39}Cl_2F_6N_2O_2]^+$: 739.2293, found 739.2289.

**Example 26**

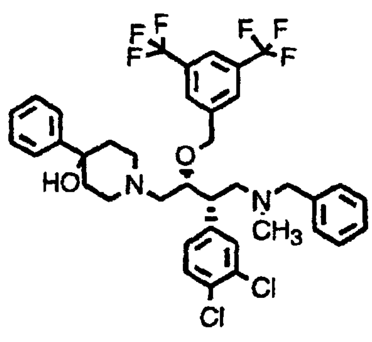

HRMS (FAB, M+H$^+$): *m/e* calc'd for $[C_{38}H_{39}Cl_2F_6N_2O_2]^+$: 739.2293, found 739.2280.

The following formulations exemplify some of the dosage forms of this invention. In each, the term "active compound" refers to a compound of formula I.

EXAMPLE A

Tablets

| No. | Ingredient | mg/tablet | mg/tablet |
|---|---|---|---|
| 1 | Active Compound | 100 | 500 |
| 2 | Lactose USP | 122 | 113 |
| 3 | Corn Starch, Food Grade, as a 10% paste in Purified Water | 30 | 40 |
| 4 | Corn Starch, Food Grade | 45 | 40 |
| 5 | Magnesium Stearate | 3 | 7 |
| | Total | 300 | 700 |

Method of Manufacture

Mix Item Nos. 1 and 2 in suitable mixer for 10-15 minutes. Granulate the mixture with Item No. 3. Mill the damp granules through a coarse screen (e.g., 1/4", 0.63 cm) if necessary. Dry the damp granules. Screen the dried granules if necessary and mix with Item No. 4 and mix for 10-15 minutes. Add Item No. 5 and mix for 1-3 minutes. Compress the mixture to appropriate size and weight on a suitable tablet machine.

EXAMPLE B

| Capsules | | | |
|---|---|---|---|
| No. | Ingredient | mg/tablet | mg/tablet |
| 1 | Active Compound | 100 | 500 |
| 2 | Lactose USP | 106 | 123 |
| 3 | Corn Starch, Food Grade | 40 | 70 |
| 4 | Magnesium Stearate NF | 4 | 7 |
| | Total | 250 | 700 |

Method of Manufacture

Mix Item Nos. 1, 2 and 3 in a suitable blender for 10-15 minutes. Add Item No. 4 and mix for 1-3 minutes. Fill the mixture into suitable two-piece hard gelatin capsules on a suitable encapsulating machine.

EXAMPLE C

| Sterile Powder for Injection | | |
|---|---|---|
| Ingredient | mg/vial | mg/vial |
| Active sterile powder | 100 | 500 |

For reconstitution add sterile water for injection or bacteriostatic water for injection.
The in *vitro* and *in vivo* activity of the compounds of formula I can be determined by the following procedures.

**In vitro procedure to identify $NK_1$ activity**

Test compounds are evaluated for their ability to inhibit the activity of the $NK_1$ agonist Substance P on the isolated guinea pig vas deferens. Freshly cut vas deferens are removed from male Hartley guinea pigs (230-350g) and suspended in 25 ml tissue baths containing Kreb's Henseleit solution warmed to 37°C and constantly aerated with

95% $O_2$ and 5% $CO_2$. Tissues are adjusted to 0.5 g and allowed to equilibrate for a period of 30 minutes. The vas deferens are exposed to an electrical field stimulation (Grass S48 Stimulator) every 60 seconds at an intensity that will cause the tissue to contract 80% of its maximum capacity. All responses are recorded isometrically by means of a Grass force displacement transducer (FT03) and Harvard electronic recorder. Substance P potentiates the electrical field stimulated-induced contractions of the guinea pig vas deferens. In unpaired studies, all tissues (control or drug treated) are exposed to cumulative concentations of Substance P ($1X10^{-10}$ M - $7X10^{-7}$ M). Single log-concentations of the test compounds are given to separate tissues and allowed to equilibrate for 30 minutes before a Substance P concentation-response curve is generated. At least 5 separate tissues are used for each control and individual drug-concentation for every drug assay.

Inhibition of the Substance P is demonstrated by a rightward shift of its concentration-response curve. These shifts are used to determine the $pA_2$ value, which is defined as the negative log of the molar concentration of the inhibitor which would require that twice as much agonist be used to elicit a chosen response. This value is used to determine relative antagonist potency.

## Isolated Hamster Trachea $NK_2$ Assay

General methodology and characterization of hamster trachea responses to neurokinin agonists as providing an $NK_2$ monoreceptor assay is found in C.A. Maggi, et al., *Eur. J. Pharmacol.* 166 (1989) 435 and J.L. Ellis, et al., *J. Pharm. Exp. Ther.* 267 (1993) 95.

Continuous isometric tension monitoring is achieved with Grass FT-03 force displacement transducers connected to Buxco Electronics preamplifiers built into a Graphtec Linearcorder Model WR 3310.

Male Charles River LAK:LVG (SYR) hamsters, 100-200 g fed weight, are stunned by a sharp blow to the head, loss of corneal reflex is assured, the hamsters are sacrificed by thoractomy and cutting the heart. Cervical trachea segments are removed to room temperature Krebs buffer, pH 7.4, aerated with 95% $O_2$ - 5% $CO_2$ gas and cleaned of adhering tissue. The segments are cut into two 3-4 mm long ring segments. Tracheal rings are suspended from transducers and anchored in 15.0 ml water jacketed organ baths by means of stainless steel hooks and 6-0 silk. Baths are filled with Krebs buffer, pH 7.4, maintained at 37°C and continuously aerated with 95% $O_2$ - 5% $CO_2$ gas. Tracheal rings are placed under 1.0 g initial tension and allowed a 90 min equilibration period with four 1 μM NKA challenge, wash and recovery cycles at 20 min intervals. 30 min vehicle pretreatment is followed by cumulative additions of rising doses of NKA (3 nM - 1 μM final concentration, 5 min intervals between additions). The final NKA response is followed by a 15 min wash and recovery period. 30 min pretreatment with a test compound or its vehicle is followed by cumulative additions of rising doses of NKA (3 nM - 10 μM final concentration if necessary, 5 min intervals between additions). The final NKA response is followed by a 1 mM carbachol challenge to obtain a maximal tension response in each tissue.

Tissue responses to NKA are recorded as positive pen displacements over baseline and converted to grams tension by comparison to standard weights. Responses are normalized as a % of the maximal tissue tension. $ED_{50}$'s are calculated for NKA from the control and treated NKA dose responses and compared. Test compounds resulting in an agonist dose ratio ≥ 2 at a screening concentration of 1 μM (i.e. $pA_2 \geq = 6.0$) are considered actives. Further dose response data is obtained for actives so that an apparent $pA_2$ estimate can be calculated. $pA_2$ is calculated either by estimation of $K_i$ as described by Furchgott (where $pA_2 = -\text{Log } K_i$, R.F. Furchgott, *Pharm. Rev.* 7 [1995] 183) or by Shild Plot Analysis (O. Arunlakshana & H.O. Shild, *Br. J. Pharmacol.* 14[1959] 48) if the data is sufficient.

## Effect of $NK_1$ Antagonists on Substance P-Induced Airway Microvascular Leakage in Guinea Pigs

Studies are performed on male Hartley guinea pigs ranging in weight from 400-650 g. The animals are given food and water *ad libitum.* The animals are anesthetized by intraperitoneal injection of dialurethane (containing 0.1 g/ml diallylbarbituric acid, 0.4 g/ml ethylurea and 0.4 g/ml urethane). The trachea is cannulated just below the larynx and the animals are ventilated ($V_T$ = 4 ml, f = 45 breaths/min) with a Harvard rodent respirator. The jugular vein is cannulated for the injection of drugs.

The Evans blue dye technique (Danko, G. et al., Pharmacol. Commun., 1, 203-209, 1992) is used to measure airway microvascular leakage (AML). Evans blue (30 mg/kg) is injected intravenously, followed 1 min later by i.v. injection of substance P (10 μg/kg). Five min later, the thorax is opended and a blunt-ended 13-guage needle passed into the aorta. An incision is made in the right atrium and blood is expelled by flushing 100 ml of saline through the aortic catheter. The lungs and trachea are removed en-bloc and the trachea and bronchi are then blotted dry with filter paper and weighed. Evans blue is extracted by incubation of the tissue at 37°C for 18 hr in 2 ml of formamide in stoppered tubes. The absorbance of the formamide extracts of dye is measured at 620 nm. The amount of dye is calculated by interpolation from a standard curve of Evans blue in the range 0.5-10 μg/ml in formamide. The dye concentration is expressed as ng dye per mg tissue wet weight. Test compounds were suspended in cyclodextran vehicle and given i.v. 5 min before substance P.

**Measurement of $NK_2$ Activity in Vivo**

Male Hartley guinea pigs (400-500 gm) with ad lib. access to food and water are anesthetized with an intraperitoneal injection of 0.9 ml/kg dialurethane (containing 0.1 g/m diallylbarbituric acid, 0.4 g/ml ethylurea and 0.4 g/ml urethane). After induction of a surgical plane of anesthesia, tracheal, esophageal and jugular venous cannulae are implanted to facilitate mechanical respiration, measurement of esophageal pressure and administration of drugs, respectively.

The guinea pigs are placed inside a whole body plethysmograph and the catheters connected to outlet ports in the plethysmograph wall. Airflow is measured using a differential pressure transducer (Validyne, Northridge CA, model MP45-1, range ± 2 $cmH_2O$) which measures the pressure across a wire mesh screen that covers a 1 inch hole in the wall of the plethysmograph. The airflow signal is electrically integrated to a signal proportional to volume. Transpulmonary pressure is measured as the pressure difference between the trachea and the esophagus using a differential pressure transducer (Validyne, Northridge, CA, model MP45-1, range ± 20 cm $H_2O$). The volume, airflow and transpulmonary pressure signals are monitored by means of a pulmonary analysis computer (Buxco Electronics, Sharon, CT, model 6) and used for the derivation of pulmonary resistance ($R_L$) and dynamic lung compliance ($C_{Dyn}$).

Bronchoconstriction Due to NKA

Increasing iv doses of NKA are administered at half log (0.01-3 µg/kg) intervals allowing recovery to baseline pulmonary mechanics between each dose. Peak bronchoconstriction occurs within 30 seconds after each dose of agonist. The dose response is stopped when $C_{Dyn}$ is reduced 80-90% from baseline. One dose-response to NKA is performed in each animal. Test compounds are suspended in cyclodextran vehicle and given i.v. 5 min before the initiation of the NKA dose response.

For each animal, dose response curves to NKA are constructed by plotting the percent increase in $R_L$ or decrease in $C_{Dyn}$ against log dose of agonist. The doses of NKA that increased $R_L$ by 100% ($R_L$100) or decreased $C_{Dyn}$ by 40% ($C_{Dyn}$40) from baseline values are obtained by log-linear interpolation of the dose response curves.

**Neurokinin Receptor Binding Assay(s)**

Chinese Hamster ovary (CHO) cells transfected with the coding regions for the human neurokinin 1 (NK1) of the human neurokinin 2 (NK2) receptors are grown in Dulbecco's minimal essential medium supplemented with 10% fetal calf serum, 0.1 mM non-essential amino acids, 2 mM glutamine, 100units/ml of penicillin and streptomycin, and 0.8 mg of G418/ml at 37°C in a humidified atmosphere containing 5% $CO_2$.

Cells are detached from T-175 flasks with a sterile solution containing 5mM EDTA in phosphate buffered saline. Cells are harvested by centrifugation and washed in RPMI media at 40°C for 5 minutes. The pellet is resuspended inTris-HCl (pH7.4) containing 1 uM phsphoramidon and 4 ug/ml of chymostatin at a cell density of 30 x $10^6$ cells/ml. The suspension is then homogenized in a Brinkman Polytron (setting 5) for 30-45 seconds. The homogenate is centrifuged at 800 x g for 5 min at 4°C to collect unbroken cells and nuclei. The supernatant is centrifuged in a Sorvall RC5C at 19,000 rpm (44,00 x g) for 30 min at 4°C. The pellet is resuspended, an aliquot is removed for a protein determination (BCA) and washed again. The resulting pellet is stored at -80°C.

To assay receptor binding, 50 µl of [$^3$H]-Substance P (9-Sar, 11-Met [02]) (specific activity 41 Ci/mmol) (Dupont-NEN) (0.8 nM for the NK-1 assay) or [$^3$H]-Neurokinin A (specific activity 114 Ci/ mmole) (Zenca) (1.0 nM for the NK-2 assay) is added to tubes containing buffer (50 mM Tris-HCl (pH 7.4) with 1 mM $MnCl_2$ and 0.2% Bovine Serum Albumin) and either DMSO or test compound. Binding is initiated by the addition of 100µl of membrane (10-20 µg) containing the human NK-1 or NK-2 receptor in a final volume of 200 µl. After 40 minutes at room temperature, the reaction is stopped by rapid filtration onto Whatman GF/C filters which have been presoaked in 0.3% polyethylenimine. Filters are washed 2 times with 3 ml of 50 mM Tris-HCl (pH7.4). Filters are added to 6 mls of Ready-Safe liquid scintillation cocktail and quantified by liquid scintillation spectrometry in a LKB 1219 RackBeta counter. Non-specific binding is determined by the addition of either 1 µM of CP-99994 (NK-1) or 1µM SR-48968 (NK-2) (both synthesized by the chemistry department of Schering-Plough Research Institute). $IC_{50}$ values are determined from competition binding curves and Ki values are determined according to Cheng and Prusoff using the experimentally determined value of 0.8 nM for the NK-1 receptor and 2.4 nM for the NK-2 receptor.

% Inhibition is the difference between the percent of maximum specific binding (MSB) and 100%. The percent of MSB is defined by the following equation, wherein "dpm" is disintegrations per minute:

$$\%\ MSB = \frac{\text{(dpm of unknown) - (dpm of nonspecific binding)}}{\text{(dpm of total binding) - (dpm of nonspecific binding)}} \times 100$$

It will be recognized that compounds of formula I exhibit $NK_1$, $NK_2$ and/or $NK_3$ antagonist activity to varying degrees, e.g., certain compounds have strong $NK_1$ antagonist activity, but weaker $NK_2$ and $NK_3$ antagonist activity, while others are strong $NK_2$ antagonists, but weaker $NK_1$ and $NK_3$ antagonists. While compounds with approximate equipotency are preferred, it is also within the scope of this invention to use compounds of with unequal $NK_1/NK_2/NK_3$ antagonist activity when clinically appropriate.

Using the test procedures described above, compounds of the present invention exhibit a range of activity: percent inhibition at a dosage of 1μM ranges from about 1 to about 81% inhibition of $NK_1$ and/or about 1 to about 96% inhibition of $NK_2$. Preferred are compounds exhibiting greater than about 50% inhibition of $NK_1$ and about 1 to about 96% inhibition of $NK_2$; also preferred are compounds exhibiting about 1 to about 81% inhibition of $NK_1$ and greater than about 50% inhibition of $NK_2$. Also preferred are compounds exhibiting greater than about 50 % inhibition of $NK_1$ and greater than about 50% inhibition of $NK_2$; of those compounds, more preferred are compounds exhibiting greater than about 75% inhibition of $NK_1$ and greater then about 75% inhibition of $NK_2$.

## Claims

**1.** A compound represented by the structural formula

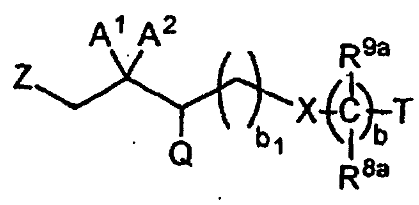

or a pharmaceutically acceptable salt thereof, wherein:

$A^1$ is $-OR^6$, $-N(R^6)(R^7)$, $-S(O)_eR^{13}$, or $-(C(R^6)(R^7))_{1-6}-N(R^6)(R^7)$ and $A^2$ is H, or $A^1$ and $A^2$ together are =O, $=C(R^6)(R^7)$, or $=NOR^6$;
Q is $R^5$-phenyl;
T is $R^4$-aryl;
b is 0, 1 or 2;
$b_1$ is 1 or 2;
X is -O-, $-NR^6-$, $-N(R^6)C(O)-$, $-OC(O)NR^6-$, or $-N(R^6)C(O)O-$;
$R^4$ and $R^5$ are independently 1-3 substituents independently selected from the group consisting of H, halogeno, $-OR^6$, $-OC(O)R^6$, $-OC(O)N(R^6)(R^7)$, $-N(R^6)(R^7)$, $C_{1-6}$ alkyl, $-CF_3$, $-C_2F_5$, $-COR^6$, $-CO_2R^6$, $-CON(R^6)(R^7)$, $-S(O)_eR^{13}$, -CN, $-OCF_3$, $-NR^6CO_2R^{16}$, $-NR^6COR^7$, $-NR^8CON(R^6)(R^7)$, $R^{15}$-phenyl, $R^{15}$-benzyl, $NO_2$, $-N(R^6)S(O)_2R^{13}$ or $-S(O)_2N(R^6)(R^7)$; or adjacent $R^4$ substituents or adjacent $R^5$ substituents can form a $-O-CH_2-O-$ group; and $R^4$ can also be $R^{15}$-heteroaryl;
$R^6$, $R^7$, $R^8$, $R^{8a}$, and $R^{13}$ are independently selected from the group consisting of H, $C_{1-6}$ alkyl, $C_2$-$C_6$ hydroxyalkyl, $C_1$-$C_6$ alkoxy-$C_1$-$C_6$ alkyl, $R^{15}$-phenyl, and $R^{15}$-benzyl; or $R^6$ and $R^7$, together with the nitrogen to which they are attached, form a ring of 5 to 6 members, wherein 0, 1 or 2 ring members are selected from the group consisting of -O-, -S- and $-N(R^{19})-$;
$R^9$ and $R^{9a}$ are independently selected from the group consisting of $R^6$ and $-OR^6$, provided that when $R^9$ is OH, X is $-N(R^6)C(O)-$;
$R^{10}$ is independently selected from the group consisting of H and $C_{1-6}$ alkyl;
$R^{15}$ is 1 to 3 substituents independently selected from the group consisting of H, $C_1$-$C_6$ alkyl, $C_1$-$C_6$ alkoxy, $C_1$-$C_6$ alkylthio, halogeno, $-CF_3$, $-C_2F_5$, $-COR^{10}$, $-CO_2R^{10}$, $-C(O)N(R^{10})_2$, $-S(O)_eR^{10}$, -CN, $-N(R^{10})COR^{10}$, $-N(R^{10})CON(R^{10})_2$ and $-NO_2$;
$R^{16}$ is $C_{1-6}$ alkyl, $R^{15}$-phenyl or $R^{15}$-benzyl;
$R^{19}$ is H, $C_1$-$C_6$ alkyl, $-C(O)N(R^{10})_2$, $-CO_2R^{10}$, $-(C(R^8)(R^9))_f-CO_2R^{10}$ or $-(C(R^8)(R^9))_u-C(O)N(R^{10})_2$;
f is 1-6;
u is 0-6; and
Z is

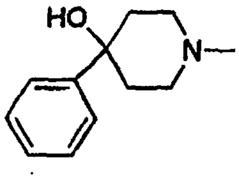 or 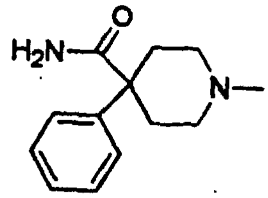 ;

wherein:

"aryl" refers to phenyl, naphthyl, indenyl, tetrahydronaphthyl, indanyl, anthracenyl or fluorenyl; and "heteroaryl" refers to 5- to 10-membered single or benzofused aromatic rings comprising 1 to 4 heteroatoms independently selected from the group consisting of -O-, -S- and -N=, provided that the rings do not include adjacent oxygen and/or sulfur atoms.

**2.** A compound according to claim 1 wherein b is 0 or 1; $b_1$ is 1; X is $-NR^6-$ or $-N(R^6)C(O)-$; $R^{8a}$ and $R^{9a}$ are independently selected from the group consisting of hydrogen, $C_2$-$C_6$ hydroxyalkyl and $C_1$-$C_6$ alkoxy-$C_1$-$C_6$-alkyl; T is phenyl substituted by two substituents selected from the group consisting of $C_1$-$C_6$ alkyl, halogeno,-$CF_3$ and $C_1$-$C_6$ alkoxy; and Q is phenyl disubstituted by halogeno.

**3.** A compound according to claim 1 which is
*N*-[2-(3,4-dichlorophenyl)-3-hydroxy-4-(4-hydroxy-4-phenyl-1-piperidinyl)butyl]-*N*-methyl benzamide;
*N*-[3-hydroxy-4-(4-hydroxy-4-phenyl-1-piperidinyl)-2-(4-methoxyphenyl)butyl]-*N*-methyl benzamide;
*N*-[3-hydroxy-4-(4-hydroxy-4-phenyl-1-piperidinyl)-2-phenylbutyl]-*N*-methyl benzamide;
*N*-[2-(4-chlorophenyl)-3-hydroxy-4-(4-hydroxy-4-phenyl-1-piperidinyl)butyl]-*N*-methyl benzamide;
*N*-[3-hydroxy-4-(4-hydroxy-4-phenyl-1-piperidinyl)-2-(4-methylphenyl)butyl]-*N*-methyl benzamide;
*N*-[2-(4-chlorophenyl)-4-(4-hydroxy-4-phenyl-1-piperidinyl)-3-methoxybutyl]-*N*-methyl benzamide;
*N*-[2-(3,4-dichlorophenyl)-4-(4-hydroxy-4-phenyl-1-piperidinyl)-3-oxobutyl]-*N*-methylbenzamide;
*N*-[2-(3,4-dichlorophenyl)-4-(4-hydroxy-4-phenyl-1-piperidinyl)-3-(methoxyimino)butyl]-*N*-methylbenzamide; or
*N*-[3-[[3,5-bis(trifluoromethyl)phenyl]methoxy]-2-(3,4-dichloro-phenyl)-4-(4-hydroxy-4-phenyl-1-piperidiny) butyl-*N*-methyl benzamide;
*N*-[3-[[3,5-bis(trifluoromethyl)phenyl]methoxy]-4-(4-hydroxy-4-phenyl-1-piperidinyl)-2-phenylbutyl]-*N*-methylbenzamide;
α-[1-(3,4-dichlorophenyl)-2-[methyl(phenylmethyl)amino]ethyl]-4-hydroxy-4-phenyl-1-piperidineethanol; or
1-[2-[[3,5-bis(trifluoromethyl)phenyl]methoxy]-3-(3,4-dichlorophenyl)-4-[methyl(phenylmethyl)amino]butyl]-4-phenyl-4-piperidinol.

**4.** A pharmaceutical composition comprising an effective amount of a compound of any preceding claim in a pharmaceutically acceptable carrier.

**5.** A method of preparing a composition of claim 4 comprising admixing a compound of any of claims 1 to 3 with pharmaceutically acceptable carrier.

**6.** Use of a compound of any of claims 1 to 3 in the manufacture of a medicament for the treatment of asthma, cough, bronchospasm, central nervous system diseases, inflammatory diseases and gastrointestinal disorders.

**Patentansprüche**

**1.** Verbindung der folgenden Strukturformel

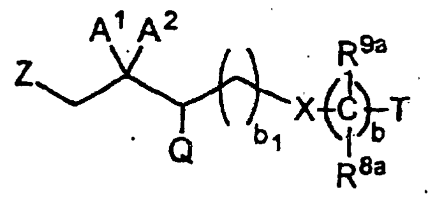

oder ein pharmazeutisch akzeptables Salz davon, bei der:

$A^1$ $-OR^6$, $-N(R^6)(R^7)$, $-S(O)_eR^{13}$ oder $-(C(R^6)(R^7))_{1-6}-N(R^6)(R^7)$ ist, und $A^2$ H ist, oder $A^1$ und $A^2$ zusammen =O, $=C(R^6)(R^7)$ oder $=NOR^6$ sind;

Q $R^5$-Phenyl ist;

T $R^4$-Aryl ist;

b 0, 1 oder 2 ist;

$b_1$ 1 oder 2 ist;

X -O-, $-NR^6-$, $-N(R^6)C(O)-$, $-OC(O)NR^6-$ oder $-N(R^6)C(O)O-$ ist;

$R^4$ und $R^5$ unabhängig 1 bis 3 Substituenten sind, die unabhängig ausgewählt sind aus der Gruppe bestehend aus H, Halogen, $-OR^6$, $-OC(O)R^6$, $-OC(O)N(R^6)(R^7)$, $-N(R^6)(R^7)$, $C_{1-6}$-Alkyl, $-CF_3$, $-C_2F_5$, $-COR^6$, $-CO_2R^6$, $-CON(R^6)(R^7)$, $-S(O)_eR^{13}$, -CN, $-OCF_3$, $-NR^6CO_2R^{16}$, $-NR^6COR^7$, $-NR^8CON(R^6)(R^7)$, $R^{15}$-Phenyl, $R^{15}$-Benzyl, $NO_2$, $-N(R^6)S(O)_2R^{13}$ oder $-S(O)_2N(R^6)(R^7)$; oder benachbarte $R^4$-Substituenten oder benachbarte $R^5$-Substituenten eine $-O-CH_2-O-$Gruppe bilden können; und $R^4$ auch $R^{15}$-Heteroaryl sein kann;

$R^6$, $R^7$, $R^8$, $R^{8a}$ und $R^{13}$ unabhängig ausgewählt sind aus der Gruppe bestehend aus H, $C_{1-6}$-Alkyl, $C_2$- bis $C_6$-Hydroxyalkyl, $C_1$- bis $C_6$-Alkoxy-$C_1$- bis $C_6$-alkyl, $R^{15}$-Phenyl und $R^{15}$-Benzyl; oder $R^6$ und $R^7$ gemeinsam mit dem Stickstoff, an den sie gebunden sind, einen Ring aus 5 bis 6 Gliedern bilden, wobei 0, 1 oder 2 Ringglieder ausgewählt sind aus der Gruppe bestehend aus -O-, -S- und $-N(R^{19})-$;

$R^9$ und $R^{9a}$ unabhängig ausgewählt sind aus der Gruppe bestehend aus $R^6$ und $-OR^6$, unter der Voraussetzung, dass, wenn $R^9$ OH ist, X $-N(R^6)C(O)-$ ist;

$R^{10}$ unabhängig ausgewählt ist aus der Gruppe bestehend aus H, und $C_1$- bis $C_6$-Alkyl;

$R^{15}$ 1 bis 3 Substituenten unabhängig ausgewählt aus der Gruppe bestehend aus H, $C_1$- bis $C_6$-Alkyl, $C_1$- bis $C_6$-Alkoxy, $C_1$bis $C_6$-Alkylthio, Halogen, $-CF_3$, $-C_2F_5$, $-COR^{10}$, $-CO_2R^{10}$, $-C(O)N(R^{10})_2$, $-S(O)_eR^{10}$, -CN, $-N(R^{10})COR^{10}$, $-N(R^{10})CON(R^{10})_2$ und $-NO_2$ ist;

$R^{16}$ $C_1$- bis $C_6$-Alkyl, $R^{15}$ Phenyl oder $R^{15}$ Benzyl ist;

$R^{19}$ H, $C_1$- bis $C_6$-Alkyl, $-C(O)N(R^{10})_2$, $-CO_2R^{10}$, $-(C(R^8)(R^9))_f-CO_2R^{10}$ oder $-(C(R^8)(R^9))_u-C(O)N(R^{10})_2$ ist;

f 1 bis 6 ist;

u 0 bis 6 ist; und

Z

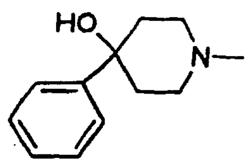 oder 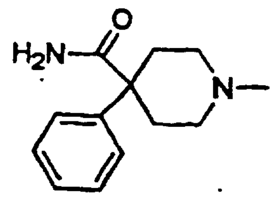

ist, wobei

"Aryl" Phenyl, Naphthyl, Indenyl, Tetrahydronaphthyl, Indanyl, Anthracenyl oder Fluorenyl bezeichnet; und "Heteroaryl" 5- bis 10-gliedrige einzelne oder benzoanellierte aromatische Ringe bezeichnet, die 1 bis 4 Heteroatome unabhängig ausgewählt aus der Gruppe bestehend aus -O-, -S- und -N= enthalten, unter der Voraussetzung, dass die Ringe nicht benachbarte Sauerstoff- und/oder Schwefelatome enthalten.

**2.** Verbindung nach Anspruch 1, bei der b 0 oder 1 ist; $b_1$ 1 ist; X -$NR^6$- oder -$N(R^6)C(O)$- ist; $R^{8a}$ und $R^{9a}$ unabhängig ausgewählt sind aus der Gruppe bestehend aus Wasserstoff, $C_2$- bis $C_6$-Hydroxyalkyl und $C_1$- bis $C_6$-Alkoxy-$C_1$- bis $C_6$-alkyl; T Phenyl ist, welches mit zwei Substituenten ausgewählt aus der Gruppe bestehend aus $C_1$- bis $C_6$-Alkyl, Halogen, -$CF_3$ und $C_1$- bis $C_6$-Alkoxy substituiert ist; und Q Phenyl ist, welches mit Halogen sustituiert ist.

**3.** Verbindung nach Anspruch 1, die
N- [2-(3,4-Dichlorphenyl)-3-hydroxy-4-(4-hydroxy-4-phenyl-1-piperidinyl)butyl]-N-methylbenzamid;
N- [3-Hydroxy-4- (4-hydroxy-4-phenyl-1-piperidinyl)-2-(4-methoxyphenyl)butyl]-N-methylbenzamid;
N- [3-Hydroxy-4- (4-hydroxy-4-phenyl-1-piperidinyl)-2-phenylbutyl]-N-methylbenzamid;
N- [2-(4-Chlorphenyl)-3-hydroxy-4-(4-hydroxy-4-phenyl-1-piperidinyl)butyl]-N-methylbenzamid;
N- [3-Hydroxy-4- (4-hydroxy-4-phenyl-1-piperidinyl) -2- (4-methylphenyl)butyl]-N-methylbenzamid;
N- [2- (4-Chlorphenyl)-4- (4-hydroxy-4-phenyl-1-piperidinyl)-3-methoxybutyl]-N-methylbenzamid;
N-[2-(3,4-Dichlorphenyl)-4-(4-hydroxy-4-phenyl-1-piperidinyl)-3-oxobutyl]-N-methylbenzamid;
N-[2-(3,4-Dichlorphenyl)-4- (4-hydroxy-4-phenyl-1-piperidinyl)-3-(methoxyimino)butyl]-N-methylbenzamid; oder
N-[3-[[3,5-Bis(trifluormethyl)phenyl]methoxy]-2-(3,4-dichlorphenyl)-4-(4-hydroxy-4-phenyl-1-piperidinyl)butyl-N-methylbenzamid;
N- [3- [[3,5-Bis(trifluormethyl)phenyl]methoxy]-4- (4-hydroxy-4-phenyl-1-piperidinyl)-2-phenylbutyl-N-methylbenzamid;
α-[1-(3,4-Dichlorphenyl)-2-[methyl(phenylmethyl)amino]-ethyl]-4-hydroxy-4-phenyl-1-piperidinylethanol; oder
1- [2- [[3,5-Bis(trifluormethyl)phenyl]methoxy]-3- (3,4-dichlorphenyl)-4-[methyl(phenylmethyl)amino]butyl]-4-phenyl-4-piperidinol
ist.

**4.** Pharmazeutische Zusammensetzung, die eine wirksame Menge einer Verbindung nach einem der vorhergehenden Ansprüche in einem pharmazeutisch akzeptablen Träger enthält.

**5.** Verfahren zur Herstellung einer Zusammensetzung gemäß Anspruch 4, bei dem eine Verbindung gemäß einem der Ansprüche 1 bis 3 mit einem pharmazeutisch akzeptablen Träger gemischt wird.

**6.** Verwendung einer Verbindung nach einem der Ansprüche 1 bis 3 zur Herstellung eines Medikaments zur Behandlung von Asthma, Husten, Bronchospasmen, Erkrankungen des zentralen Nervensystems, Entzündungserkrankungen und gastrointestinalen Störungen.

## Revendications

**1.** Composé représenté par la formule structurale

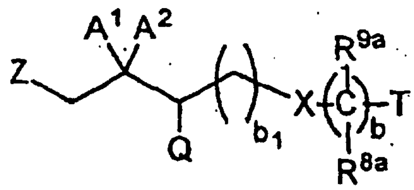

ou sel d'un tel composé, admissible en pharmacie, dans laquelle formule :

$A^1$ représente $-OR^6$, $-N(R^6)(R^7)$, $-[C(R^6)(R^7)]_{1-6}-N(R^6)(R^7)$ ou $-S(O)_eR^{13}$ et $A^2$ représente -H, ou bien $A^1$ et $A^2$ représentent conjointement =O, $=C(R^6)(R^7)$ ou $=NOR^6$ ;
Q représente $R^5$-phényle ;
T représente $R^4$-aryle ;
b vaut 0, 1 ou 2 ;
$b_1$ vaut 1 ou 2 ;
X représente -O-, $-NR^6-$, $-N(R^6)C(O)-$,$-OC(O)NR^6-$ ou $-N(R^6)C(O)O-$ ;
$R^4$ et $R^5$ représentent chacun, indépendamment, 1 à 3 substituants choisis indépendamment dans l'ensemble formé par les atomes d'hydrogène et d'halogène et les groupes $-OR^6$, $-OC(O)R^6$, $-OC(O)N(R^6)(R^7)$, $-N(R^6)(R^7)$, alkyle en $C_{1-6}$, $-CF_3$, $-C_2F_5$, $-COR^6$, $-CO_2R^6$, $-C(O)N(R^6)(R^7)$, $-S(O)_eR^{13}$, -CN, $-OCF_3$, $-NR^6CO_2R^{16}$, $-NR^6COR^7$, $-NR^8CON(R^6)(R^7)$, $R^{15}$-phényle, $R^{15}$-benzyle, $-NO_2$, $-N(R^6)S(O)_2R^{13}$ ou $-S(O)_2N(R^6)(R^7)$, ou des substituants $R^4$ adjacents, ou des substituants $R^5$ adjacents, peuvent former un groupe $-O-CH_2-O-$, et $R^4$ peut aussi représenter un groupe $R^{15}$-hétéroaryle ;
$R^6$, $R^7$, $R^8$, $R^{8a}$ et $R^{13}$ sont choisis, indépendamment, dans l'ensemble formé par l'atome d'hydrogène et les groupes alkyle en $C_{1-6}$, hydroxyalkyle en $C_{2-6}$, (alcoxy en $C_{1-6}$)-alkyle en $C_{1-6}$, $R^{15}$-phényle et $R^{15}$-benzyle, ou bien $R^6$ et $R^7$, conjointement avec l'atome d'azote auquel ils sont rattachés, représentent un cycle à 5 ou 6 chaînons, dont 0, 1 ou 2 chaînons sont choisis parmi -O-, -S- et $-N(R^{19})-$ ;
$R^9$ et $R^{9a}$ sont choisis, indépendamment, dans l'ensemble formé par $-R^6$ et $-OR^6$, sous réserve que, si $R^9$ représente -OH, X représente $-N(R^6)C(O)-$ ;
$R^{10}$ est choisi, indépendamment, dans l'ensemble formé par l'atome d'hydrogène et les groupes alkyle en $C_{1-6}$ ;
$R^{15}$ représente 1 à 3 substituants choisis indépendamment dans l'ensemble formé par les atomes d'hydrogène et d'halogène et les groupes alkyle en $C_{1-6}$, alcoxy en $C_{1-6}$, alkylthio en $C_{1-6}$, $-CF_3$, $-C_2F_5$, $-COR^{10}$, $-CO_2R^{10}$, $-C(O)N(R^{10})_2$, $-S(O)_eR^{10}$, -CN, $-NR^{10}COR^{10}$, $-NR^{10}CON(R^{10})_2$ et $-NO_2$ .
$R^{16}$ représente un groupe alkyle en $C_{1-6}$, $R^{15}$-phényle ou $R^{15}$-benzyle ;
$R^{19}$ représente un atome d'hydrogène ou un groupe alkyle en $C_{1-6}$, $-C(O)N(R^{10})_2$, $-CO_2R^{10}$, $-[C(R^8)(R^9)]_f-CO_2R^{10}$ ou $-[C(R^8)(R^9)]_u-C(O)N(R^{10})_2$ ;
f vaut de 1 à 6 ;
u vaut de 0 à 6 ;
et Z représente

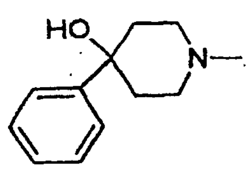

ou

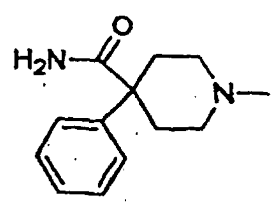

;

étant entendu

que le terme "aryle" désigne ici un groupe phényle, naphtyle, indényle, tétrahydronaphtyle, indanyle, anthracényle ou fluorényle ;

et que le terme "hétéroaryle" désigne ici un groupe cyclique aromatique simple ou benzo-condensé, de 5 à 10 chaînons, et comportant de 1 à 4 hétéroatomes choisis parmi -O-, -S- et -N=, sous réserve que ce cycle ne contiennent pas d'atomes d'oxygène et/ou de soufre situés en positions adjacentes.

**2.** Composé conforme à la revendication 1, dans lequel b vaut 0 ou 1, $b_1$ vaut 1, X représente $-NR^6-$ ou $-N(R^6)C(O)-$,

$R^{8a}$ et $R^{9a}$ sont choisis, indépendamment, dans l'ensemble formé par l'atome d'hydrogène et les groupes hydroxyalkyle en $C_{2-6}$ et (alcoxy en $C_{1-6}$)-alkyle en $C_{1-6}$, T représente un groupe phényle portant deux substituants choisis dans l'ensemble formé par les atomes d'halogène et les groupes alkyle en $C_{1-6}$, $-CF_3$ et alcoxy en $C_{1-6}$, et Q représente un groupe phényle portant deux substituants halogéno.

**3.** Composé conforme à la revendication 1, qui est l'un des suivants :

N-[2-(3,4-dichlorophényl)-3-hydroxy-4-(4-hydroxy-4-phényl-1-pipéridinyl)-butyl]-N-méthyl-benzamide ;
N-[3-hydroxy-4-(4-hydroxy-4-phényl-1-pipéridinyl)-2-(4-méthoxyphényl)-butyl]-N-méthyl-benzamide ;
N-[3-hydroxy-4-(4-hydroxy-4-phényl-1-pipéridinyl)-2-phényl-butyl]-N-méthyl-benzamide ;
N- [2-(4-chlorophényl)-3-hydroxy-4-(4-hydroxy-4-phényl-1-pipéridinyl)-butyl]-N-méthyl-benzamide ;
N-[3-hydroxy-4-(4-hydroxy-4-phényl-1-pipéridinyl)-2-(4-méthylphényl)-butyl]-N-méthyl-benzamide ;
N-[2-(4-chlorophényl)-4-(4-hydroxy-4-phényl-1-pipéridinyl)-3-méthoxybutyl]-N-méthyl-benzamide ;
N-[2-(3,4-dichlorophényl)-4-(4-hydroxy-4-phényl-1-pipéridinyl)-3-oxobutyl]-N-méthyl-benzamide ;
N-[2-(4-chlorophényl)-4-(4-hydroxy-4-phényl-1-pipéridinyl)-3-(méthoxyimino)-butyl]-N-méthyl-benzamide ;
N-{3-[(3,5-bis(trifluorométhyl)phényl)méthoxy]-2-(3,4-dichlorophényl)-4-(4-hydroxy-4-phényl-1-pipéridinyl)-butyl}-N-méthyl-benzamide ;
N-{3-[(3,5-bis(trifluorométhyl)phényl)méthoxy]-4-(4-hydroxy-4-phényl-1-pipéridinyl)-2-phényl-butyl}-N-méthyl-benzamide ;
α-{1-(3,4-dichlorophényl)-2-[méthyl(phénylméthyl)amino]-éthyl}-4-hydroxy-4-phényl-1-pipéridine-éthanol ;
1- {2-[(3,5-bis(trifluorométhyl)phényl)méthoxy]-3-(3,4-dichlorophényl)-4-[méthyl(phénylméthyl)amino]-butyl}-4-phényl-4-pipéridinol.

**4.** Composition pharmaceutique comprenant, en une quantité efficace, un composé conforme à l'une des revendications précédentes, au sein d'un véhicule admissible en pharmacie.

**5.** Procédé de préparation d'une composition conforme à la revendication 4, qui comporte le fait de mélanger un composé conforme à l'une des revendications 1 à 3 avec un véhicule admissible en pharmacie.

**6.** Emploi d'un composé conforme à l'une des revendications 1 à 3 dans la fabrication d'un médicament destiné au traitement de l'asthme, de la toux, des spasmes bronchiques, des maladies du système nerveux central, des maladies inflammatoires et des troubles gastro-intestinaux.